# EUROPÄISCHE PATENTSCHRIFT

(11) **EP 0 727 436 B1**
(45) Veröffentlichungstag und Bekanntmachung des Hinweises auf die Patenterteilung: **03.06.1998**
(21) Anmeldenummer: 96100443.9
(22) Anmeldetag: 21.03.1990
(51) Int. Cl.: C07K 16/30, A61K 39/395, A61K 47/48, A61K 49/00

(54) **Monoklonale Antikörper (MAK) gegen tumorassoziierte Antigene, ihre Herstellung und Verwendung**
Monoclonal antibodies against tumor associated antigens, their preparation and use
Anticorps monoclonaux contre les antigènes associés aux tumeurs, leur préparation et utilisation

(30) Priorität: 24.03.1989 DE 3909799
(43) Veröffentlichungstag der Anmeldung: 21.08.1996
(62) Teilanmeldung aus: 90105322.3
(73) Patentinhaber: BEHRINGWERKE Aktiengesellschaft, 35001 Marburg (DE)
(72) Erfinder: Bosslet, Klaus, Dr., 35041 Marburg (DE); Seemann, Gerhard, Dr., 35041 Marburg-Elnhausen (DE); Sedlacek, Hans-Harald, Prof.Dr., 35041 Marburg (DE); Auerbach, Bernhard, Dr., 35041 Marburg (DE)

(56) Entgegenhaltungen:
- EP-A- 0 141 079
- EP-A- 0 268 468
- EP-A- 0 368 684

## Beschreibung

Die Erfindung betrifft den murinen monoklonalen Antikörper (MAK), A, der gegen ein tumorassoziiertes Antigen gerichtet ist. Die annähernd vollständigen Nukleotidsequenzen der V-Gene dieses MAK werden beschrieben, so daß die betreffenden variablen Domänen zu chimären MAK zusammensetzbar sind bzw. durch Einsetzen der hypervariablen Regionen ("Complementarity Determining Regions = CDR) in ein humanes MAK-Gerüst "humanisierte" MAK erhalten werden. Solche Antikörperkonstrukte sind ohne die bei murinen MAK beobachteten Nachteile in der Humantherapie und in vivo Diagnostik einsetzbar.

Die verschiedentlich in der Anmeldung erwähnten Antikörper MAK B, C und D sind nicht Teil des Anspruchsbegehrens.

MAK A reagiert gegen ein Antigen 3, MAK B gegen ein Antigen 11 und MAK C gegen ein Antigen 7, die jeweils in EP-A2-0 141 079 beschrieben sind und stellen membran-assoziierte Antigene dar auf permanenten humanen Tumorzellinien, wie die CaLu-1, Chago-, Oat 75-, PaTuII- und Bewo-Zellinie. MAK D ist gegen ein Vibrio cholerae Neuraminidase (VCN)-resistentes Epitop auf dem Gangliosid GD₂ gerichtet, das auf humanen Melanomzellinien exponiert ist.

MAK A bis D wurden, wie in EP-A2-0 141 079 beschrieben, erzeugt und nach Standardmethoden isoliert.

MAK A bindet an Zellen des Granulozyten-Kompartments und an Kolon-, Pankreas- sowie einige Lungen- und Mamma-Karzinome wie in Tab. 1 dargestellt.

**Tab. 1**

| Bindungsverhalten von MAK A | | |
|---|---|---|
| untersuchte maligne Gewebsproben | Anzahl positive | Gesamtzahl |
| colorektale Karzinome: | | |
| primäre Karzinome | 6 | 6 |
| Leber-Metastasen | 15 | 16 |
| Pankreas-Karzinome | 6 | 8 |

| Lungen-Karzinome: | | |
|---|---|---|
| klein-zellige | 1 | 2 |
| adeno | 9 | 10 |
| Plattenepithel | 3 | 4 |
| groß-zellige | 1 | 2 |
| Mamma-Karzinom | 1 | 3 |

MAK B bindet an fast alle Karzinome des Gastrointestinaltrakts sowie an einige Ovar-Karzinome und Adenokarzinome der Lunge, während er mit der Mehrzahl von normalen humanen Geweben nicht bzw. dort nur mit Sekret-haltigen Stellen reagiert. Eine Übersicht über das Bindungsverhalten ist in Tab. 2 dargestellt.

**Tab. 2**

| Bindungsverhalten von MAK B | | |
|---|---|---|
| untersuchte maligne Gewebsproben | Anzahl positive | Gesamtzahl |
| colorektale Karzinome: | | |
| primäre | 6 | 6 |
| Leber-Metastasen | 9 | 10 |
| Pankreas-Karzinome | 5 | 6 |
| Magen-Karzinome | 4 | 4 |

| Lungen-Karzinome: | | |
|---|---|---|
| klein-zellige | 2 | 11 |
| adeno | 9 | 10 |
| Plattenepithel | 2 | 12 |
| groß-zellige | 4* | 12 |
| Mamma-Karzinom | 2 | 9 |
| Ovarielle (Sekret-haltige Karzinome Stellen) | 4 | 6 |
| Nieren-Karzinome | 1 | 12 |

| | | |
|---|---|---|
| * schwache, heterogene Reaktion | | |

MAK C zeigt eine deutliche Reaktion mit 70-80% der Pankreaskarzinomprimärtumore des Stadiums I und II (11/14). Pankreaskarzinommetastasen vom Grad I und II scheinen ebenso wie die Primärtumore in ihrer Mehrzahl positiv zu reagieren (3/4). Der Reaktionstyp auf diesen positiven Geweben deutet darauf hin, daß das von MAK C erkannte Epitop im Zytoplasma, auf der Membran und im interzellulären Raum lokalisiert ist.

Pankreaskarzinome des Grades III exprimieren das Epitop nicht (Primärtumor 0/2, Metastasen 0/5). Da aber die Pankreaskarzinome vom Grad I und II bis zu 95% der Pankreaskarzinome ausmachen, sollte MAK C mit 60-70% aller Pankreaskarzinome reaktiv sein.

Eine weitere wichtige Eigenschaft des MAK C ist seine Reaktivität mit dem Gangsystem im chronisch entzündeten Pankreas (Pankreatitis 10/13), wohingegen keine Bindung an gesundes exokrines und endokrines Pankreasgewebe nachweisbar ist (0/8). Von den untersuchten Colonkarzinomprimärtumoren war nur eine Minorität reaktiv (4/14), wohingegen die Mehrzahl der Colonkarzinom-Lebermetastasen eine deutliche Bindung zeigte (7/10).

Kreuzreaktivitäten des MAK C sind auf die Mucus-produzierenden Becherzellen der Colon-Mucosa und des Magens beschränkt. Alle anderen getesteten Normalgewebe, inklusiv peripherer Blutleukozyten und Knochenmark, exprimieren das von MAK C erkannte Mucus-assoziierte Epitop nicht (siehe Tab. 3).

MAK C erkennt ein Epitop auf einem tumorassoziierten Antigen, das im Serum von Patienten mit gastrointestinalen Tumoren wie z. B. dem Pankreaskarzinom in erhöhter Konzentration nachgewiesen und damit als Tumormarker genutzt werden kann.

**Tab. 3**

| Übersicht über die mit MAK C (indirekte Immunperoxidase) untersuchten Formaldehyd-fixierten, Paraffin-eingebetteten Humangewebe. | | |
|---|---|---|
| Pankreastumore | Grad I/II | Grad III |
| Primärkarzinom | 11/14 | 0/2 |
| Karzinomlebermetastase | 3/4 | 0/5 |
| Papillenkarzinom | 1/2 | |
| Cystadenom | 0/1 | |

| Pankreasgewebe | | |
|---|---|---|
| durch Pankreatitis veränderte Gänge | 10/13 | |
| normales exo- und endokrines Pankreas | 0/8 | |

| Colonkarzinome | | |
|---|---|---|
| Primärtumore | 4/14 | |
| Lebermetastasen | 7/10 | |

| Colonnormalgewebe | | |
|---|---|---|
| Becherzellen der Mucosa, luminaler Teil | 9/10 | |
| zur muscularis hingewandter Teil der Mucosa | 3/10 | |
| Muscularis mucosae | 0/7 | |
| Submucosa | 0/7 | |
| Tunica muscularis | 0/7 | |
| Lungenkarzinome | 8/18 (einige wenige Tumorzellen) | |
| Mammakarzinome | 0/3 | |

| Sonstige untersuchte Normalgewebe | | |
|---|---|---|
| Lunge | 0/3 | |
| Leber | 0/5 | |
| Mamma | 0/1 | |
| Magen Muskulatur | 0/2 | |
| Mucus-prod. Zellen | 3/3 | |
| Niere | 0/5 | |
| Lymphknoten | 0/4 | |
| Milz | 0/2 | |
| Knochenmark | 0/1 | |
| periphere Blutleukozyten | 0/2 | |
| Bindegewebe | 0/2 | |
| Muskulatur | 0/10 | |

MAK D erkennt ein VCN-sensitives Epitop auf dem Gangliosid GD₂, das nicht auf anderen bovinen Zerebral-Gangliosiden einschließlich GD₃, GM₃, GM₁, GT₁ₐ, GD_{1b}, GD₁ₐ, und GM₄ vorkommt. Mittels MAK D konnten alle Gliome, Meningiome, Neurilemmome im normalen humanen Zerebral-Gewebe angefärbt werden. In der Tab. 4 sind die Bindungseigenschaften von MAK bezüglich intrakranialer Tumoren zusammengefaßt.

**Tab. 4**

| Tumor-Bezeichnung | Anzahl der Tumore | |
|---|---|---|
| | positiv | Gesamtzahl |
| gut differenzierte Gliome, Grad I-II | 6 | 6 |
| Maligne Gliome, Grad III-IV | 10 | 10 |
| Meningiome | 11 | 11 |
| Neurilemmome | 4 | 4 |
| Zirbeldrüsen-Adenome | 0 | 5 |
| Metastasen von Karzinomen | 0 | 1 |

Daneben ist das durch MAK D definierte Epitop auf Neuroblastomen, Ganglioneuroblastomen und Ganglioneuromen anwesend. Eine Übersicht zeigt Tab. 5.

**Tab. 5**

| Reaktivität von MAK D mit Neuroblastomen und kleinen rundzelligen Tumoren des Kindes | | |
|---|---|---|
| Neuroblastome | Anzahl der Tumore | |
| | positiv | Gesamtzahl |
| Grad I | 1 | 1 |
| Grad II | 6 | 6 |
| Grad III | 4 | 4 |
| Ganglioneuroblastome | 3^{xx} | 3 |
| Ewing Sarcome | 0 | 2 |
| Rhabdomyosarcome | 0 | 1 |
| Non-Hodgkin Lymphome | 2^{xxx} | 5 |

| | | |
|---|---|---|
| ^{x} Einteilung nach Hughes | | |
| ^{xx} Ganglion Zellen | | |
| ^{xxx}einige positive Tumorzellen | | |

MAK D ist deshalb geeignet zur Differentialdiagnose von Neuroblastomen und kleinen rundzelligen Tumoren bei Kindern.

Die Spezifität von MAK D gegenüber zwei weiteren vom Neuroectoderm abgeleiteten Tumoren, nämlich Melanom- und kleinzelligen Lungenkarzinomen (SCLC) sowie gegen nicht verwandte Tumoren zeigt Tab. 6.

**Tab. 6**

| | Anzahl der Tumoren | |
|---|---|---|
| | positiv | Gesamtzahl |
| Melanoma | 4* | 10 |
| SCLC | 2* | 11 |
| Colon Karzinome | 0 | 3 |
| Mamma-Karzinome nicht SCLC: | 0 | 3 |
| Adeno Karzinome | 0 | 3 |
| Plattenepithel-Lungenkarzinome | 0 | 3 |
| großzellige Karzinome | 0 | 3 |

| | | |
|---|---|---|
| * schwache und heterogene Färbung einiger weniger Tumorzellen | | |

Mittels der in den Beispielen aufgeführten Methodik wurden die Immunglobulin V-Gene von MAK A bis MAK D isoliert.

Die Nukleotid- bzw. Proteinsequenz zeigen die Tabellen 7 (V-Gen von MAK A), 8 (V-Gen von MAK B), 9 (V-Gen von MAK C) und 10 (V-Gen von MAK D). Die CDR's sind dabei nach Kabat und Wu (a.a.O., s. Beispiele S.11) identifizierbar.

Die Erfindung betrifft folglich den murinen monoklonalen Antikörper (MAK) A, sowie monoklonale Antikörper, die dessen V-Gene oder Teile davon (Complementarity Determining Regions) enthalten, wobei ein humanes Antikörpergerüst bzw. Gerüstteile bevorzugt sind. Ferner betrifft die Erfindung Konstrukte, die dessen V-Gene oder Teile davon zusammen mit Enzymen oder radioaktiven Markierungen oder Toxinen oder katalytischen Antikörpern oder Kombinationen verschiedener V-Gen-Spezifitäten oder MHC-Klasse I oder Klasse II Antigenen oder cytolytischen Komponenten enthalten. Schließlich betrifft die Erfindung Arzneimittel, die die vorgenannten MAK enthalten und die Verwendung dieser MAK zur in vitro oder in vivo Diagnostik.

Die Erfindung ist ferner in den Patentansprüchen enthalten.

### Beispiele:

Im folgenden wurde die Polymerase-Kettenreaktion (PCR) nach Saiki et al., Science 230, 1350-1354 (1985) für die Klonierung und Expression der variablen Domäne von murinen Immunglobulinen (Ig) eingesetzt.

### 1. Identifizierung der konservierten Regionen am 5' und am 3' Ende der murinen schweren Ig-Kette (VH) und der leichten Ig-Kette (VK)

Aus der Datei von Kabat et al. "Sequences of Proteins of Immunological Interest, US Dept. of Health and Human Services, US Government Printing Office (1987) wurden die aufeinander ausgerichteten Sequenzen der variablen Regionen entnommen. Die Nukleotid-Sequenzen beginnen dort am Amino-Terminus des reifen Proteins und schließen nicht die Signalsequenzen mit ein. Per Computer-Screening (DBUTIL, R. Staden (1986) Nucleic Acids Res. 14, 217-231) wurden geeignete Primer zur cDNA-Synthese bzw. Amplimere zum Einsatz in der PCR gefunden:

Oligonukleotid I:

Bst E11 VH1FORWARD
5' TGA GGA GAC GGT GAC CGT GGT CCC TTG GCC CCA G 3'

Oligonukleotid II:

CKI
5' TGC AGC ATC AGCC 3'

Oligonukleotid III:

5' AG GTC CAG CTG CAG GAG TCT GG 3'
G A A C A

PstI
VH1 BACKWARD

Oligonukleotid IV:

5' GAC ATT CAG CTG ACC CAG TCT CCA 3'
PvuII
VKI BACKWARD

Oligonukleotid V:

VKIFORWARD
5' GTT AGA TCT CCA GCT TGG TCC C 3'
Bgl II

### 2. cDNA-Synthese

Aus ca. 3 x 10⁸ Zellen der jeweiligen Hybridome, die MAK A, B, C oder D sezernieren, wurde RNA präpariert und aus dieser mit Hilfe von Oligo dT Sepharose poly A+ mRNA angereichert. Die poly A+ mRNA wurde zur cDNA Synthese eingesetzt. Die ersten Stränge cDNA wurden mit Hilfe von Oligonukleotidprimern synthetisiert, die in der J-Region der V_{H} Nukleotidsequenzen (Oligonukleotid I) und am 5' Ende der Kappa C-Gen Nukleotidsequenzen (Oligonukleotid II) hybridisieren.

Die RNA wird dann durch NaOH Behandlung zerstört. Die Synthese der zweiten Stränge cDNA erfolgte unter Verwendung von Oligonukleotidprimern, die an den 5' Enden der V_{H} (Oligonukleotid III) und der V_{Kappa} (Oligonukleotid IV) Nukleotidsequenzen hybridisieren.

### 3. Amplifikation der synthetisierten cDNA und Sequenzierung der variablen Domänen

Die wie bei 2. beschrieben erzeugte DNA wurde mit Hilfe der Oligonukleotide I, III, IV und V (Oligonukleotid V hybridisiert in der J-Region der V_{Kappa} Nukleotidsequenzen) und der Taq DNA Polymerase aus Thermophilus Aquatius amplifiziert Ein typischer Ansatz enthielt in 50 µl Gesamtvolumen 5 µl ds DNA (hergestellt in 2.), 25 pmol Amplimere, 250 µM jeweils dATP, dTTP, dCTP und DGTP, 67 mM Tris-Hcl pH 8.8, 17 mM (NH₄) SO₄, 10 mM Mg Cl₂, 200 µg/ml Gelatine und 2 Einheiten Taq-Polymerase. Der Ansatz wurde mit Paraffinöl überschichtet und anschließend wurden 25 Zyklen von jeweils 1 Min. bei 95°C (zum Denaturieren), 1 Min. bei 30°C (Hybridisierung) und 2 Min. bei 72°C (DNA-Synthese) mittels eines Techne PHC-1 programmierbaren Heizblocks durchgeführt.

Die für die cDNA Klonierung und Amplifikation verwendeten Oligonukleotide enthalten Restriktionsschnittstellen. Durch die cDNA Klonierung und die Amplifikation wurden diese Restriktionsschnittstellen am 5' Ende und am 3' Ende der V_{H} und V_{Kappa} Nukleotidsequenzen eingeführt (Pst I und BstEII in V_{H} und Pvu II und BglII in V_{Kappa}).
Diese Restriktionsschnittstelen wurden dann dazu verwendet, die V_{H} und V_{K} cDNA Fragmente in M13 Vektoren (Lys 19, Lys 17) (Verhoyen et al. Science 239, (1988), 1534-1536) zu klonieren.
Schließlich wurden die Nukleotidsequenzen der jeweiligen V_{H} und V_{Kappa} cDNA Fragmente mit der Methode von Sanger (PNAS, USA, 74, 5463-5467, (1977)) aus den Lys 19 und Lys 17 Vektoren bestimmt (siehe Tab. 7, 8, 9, 10).

### Beispiel 4:

Der MAK C wurde adsorptiv an die Wandung von Vertiefungen von Mikrotitrationsplatten (Nunc) gebunden. In die so präparierten Vertiefungen wurden zu je 100 µl Puffer (OSND, Behringwerke (BW)) je 20 µl Probe hinzupipettiert und 2 h bzw. 3 h bei 37°C inkubiert. Nach dreimaligem Waschen mit verdünntem Enzygnost-Waschpuffer (OSEW, BW) wurden in die einzelnen Vertiefungen je 100 µl Konjugatlösung eingefüllt. Zur Anwendung kamen Konjugate des Enzyms Peroxidase sowohl mit Lektinen (z. B. : wheat germ agglutinin = WGA) als auch mit anderen Antikörpern, die weitere Epitope auf den vom MAK C definierten, tumorassoziierten Antigenen erkannten. Der folgende zweistündige bzw. dreistündige Inkubationsschritt bei 37°C wurde mit einem dreimaligen Waschzyklus abgeschlossen.

Für den dritten Inkubationsschritt bei Raumtemperatur wurden anschließend je 100 µl einer Puffer/Substrat-Chromogenlösung (OUVG/OUVF, BW) in die Vertiefungen pipettiert und die Enzymreaktion nach 30 min mit 100 µl Enzygnost-Stopplösung (OSFA, BW) beendet. Die Extinktion der Proben wurde bei 450 nm ermittelt.

### Ergebnis:

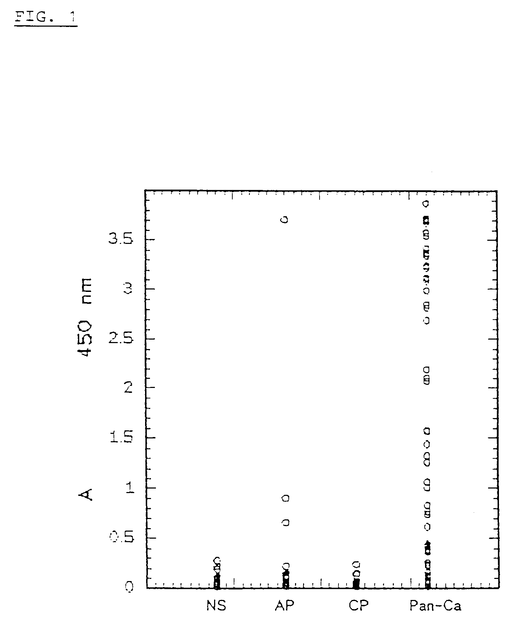

Die so ermittelten Extinktionswerte entsprechen in ihrer Höhe der Konzentration der/des Antigene(s) in den Proben. Die Konzentration des durch die spezifische Bindung des MAK C definierten Antigens im Serum oder Plasma von Patienten mit malignen Tumoren ist im Vergleich zu der bei gesunden Kontrollpersonen oder Patienten mit benignen Erkrankungen deutlich erhöht. Dies gilt vor allem für Patienten mit einem Pankreaskarzinom (Fig. 1). Deutlich erhöhte Konzentrationen wurden auch besonders im Serum oder Plasma von Patienten mit einem Magen-, Colon- oder Rectumkarzinom ermittelt. Unabhängig vom Konjugatsystem - ob Antikörper-Peroxidase (z. B. Fig. 1) oder WGA-Peroxidase - wurden vergleichbar gute Ergebnisse erzielt. Mit dem Einsatz des MAK C als spezifischem Bindungspartner läßt sich ein sensitiver Tumormarkertest für maligne und besonders für gastrointestinale Tumorerkrankungen aufbauen.

### Beispiel 5:

Je 5 µg des MAK C bzw. als Kontrolle 5 µg des MAK C50 (Pharmacia: Holmgren et al., British Medical Journal 288: 1479, (1984)) wurden in 50 µl PBS zu einem CA 19-9-Testansatz (CA 19-9 EIA "Roche") mit dem höchsten Standard (100 U/ml) vor Inkubationsbeginn hinzupipettiert. Nach Beendigung des Testes entsprechend der Originaltestvorschrift wurde die Extinktion der einzelnen Proben bestimmt.

### Ergebnis:

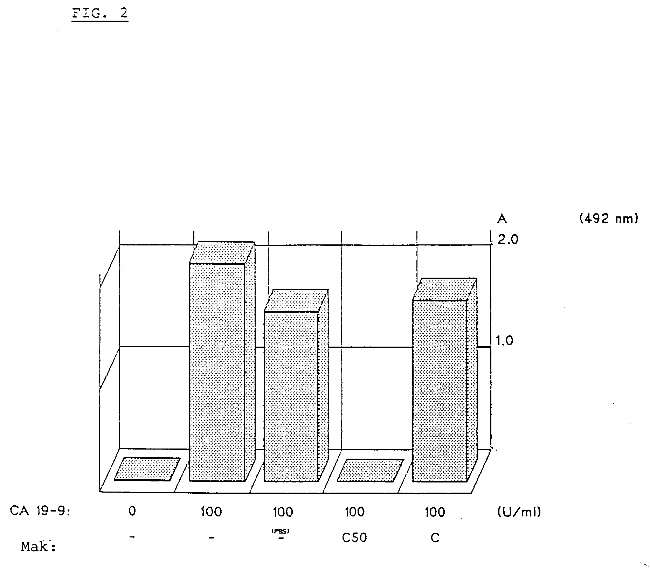

Durch die zusätzliche Verdünnung des Standard-Antigens nach Zugabe der PBS-Lösung wird auch ohne MAK-Zusatz das Signal des höchsten Standards geringfügig reduziert. Kein weiterer Signalabfall erfolgt durch die Anwesenheit des MAK C im Testansatz. Im Gegensatz hierzu wird die Signalbildung durch den MAK C50, der bekanntermaßen unter anderem auch an das vom MAK 19-9 erkannte Epitop "Sialosyl Le^{a}" spezifisch bindet, völlig unterdrückt (Fig. 2.) Hieraus kann gefolgert werden, daß der MAK C ein anderes Epitop erkennt als der MAK 19-9.

### Legende zu Fig. 1:

NS bedeutet Serum-Proben von gesunden Blutspenden (n=141); AP bedeutet Serum-Proben von Patienten mit akuter Pankreatitis (n=56); CP bedeutet Serum-Proben von Patienten mit chronischer Pankreatitis (n=40) und Pan-Ca bedeutet Patienten mit Pankreaskarzinom (n=82); A bedeutet Extinktion

### Legende zu Fig. 2:

Siehe Fig. 1; (A 19-9 bedeutet MAK CA 19-19).

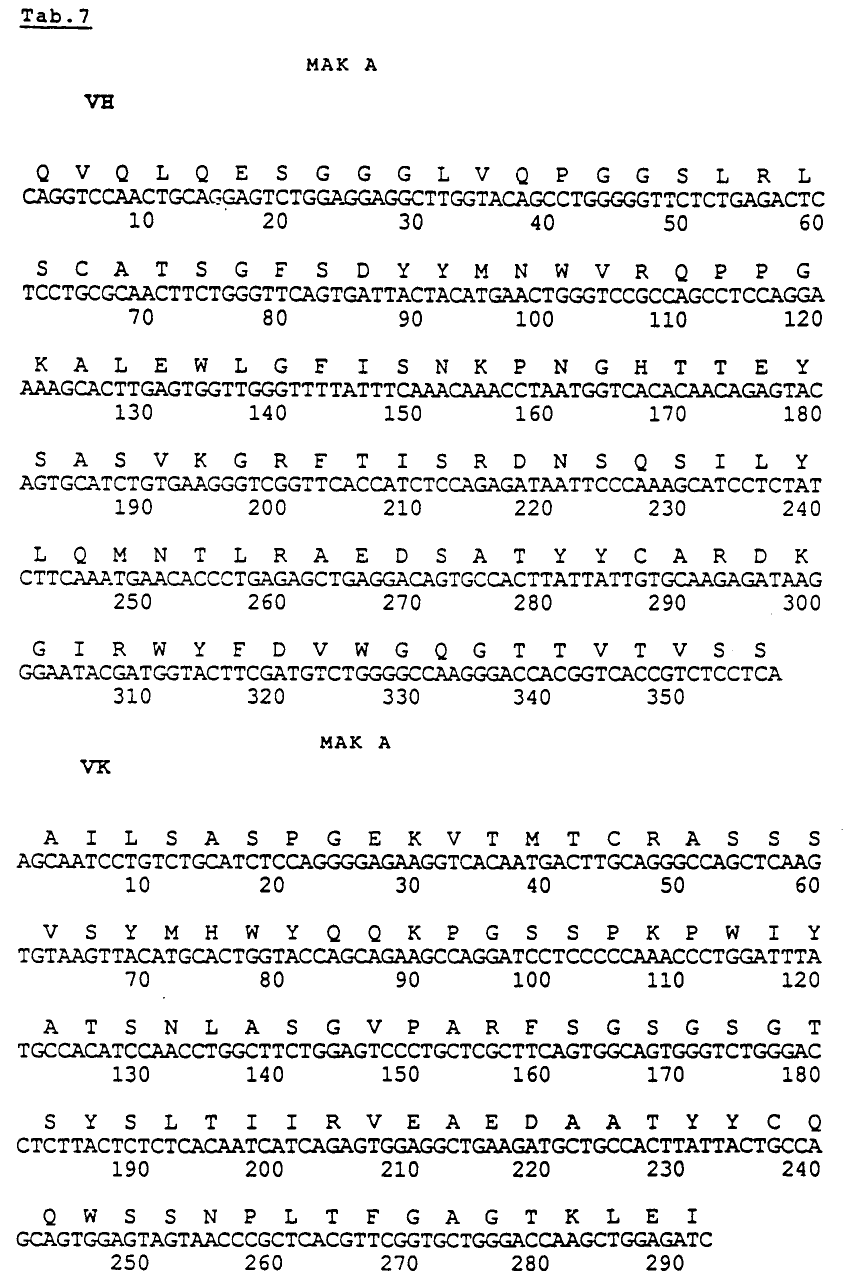

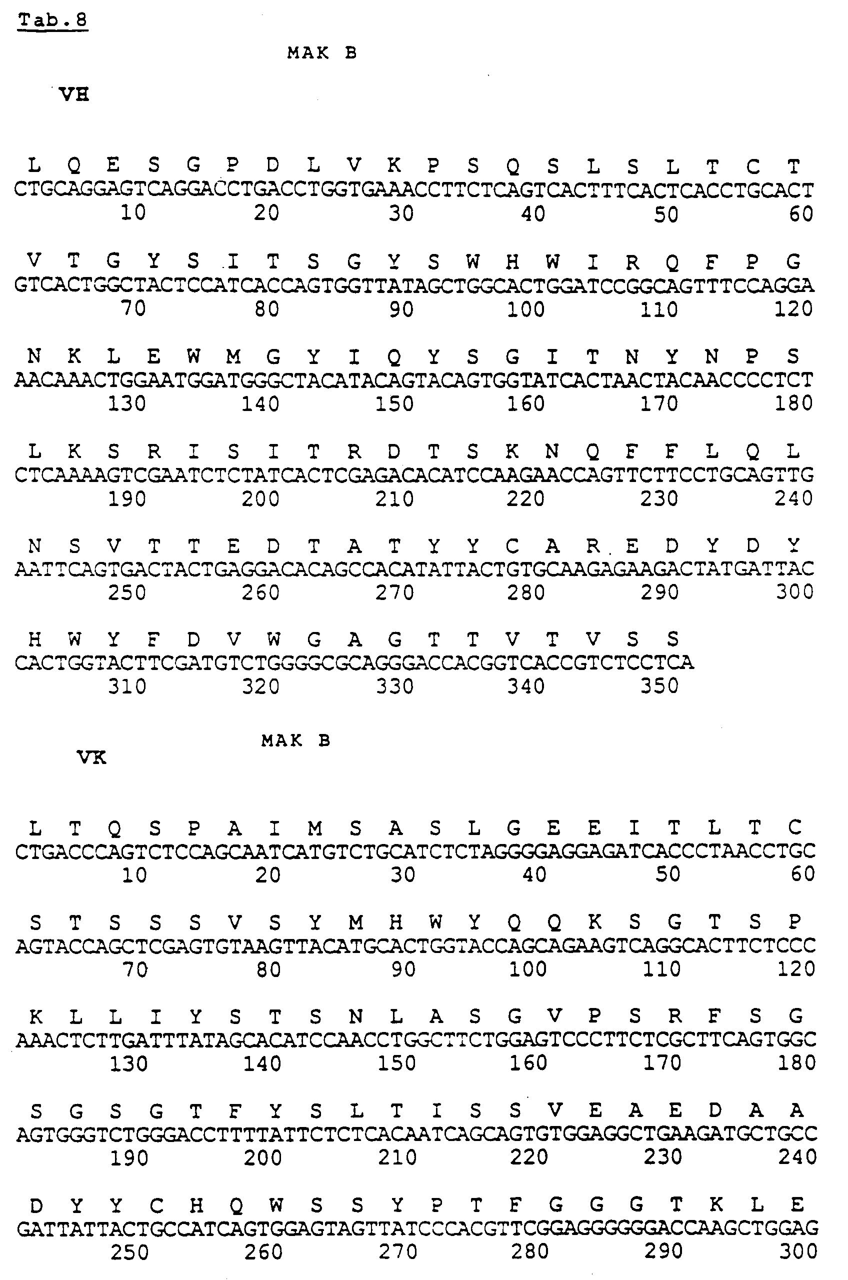

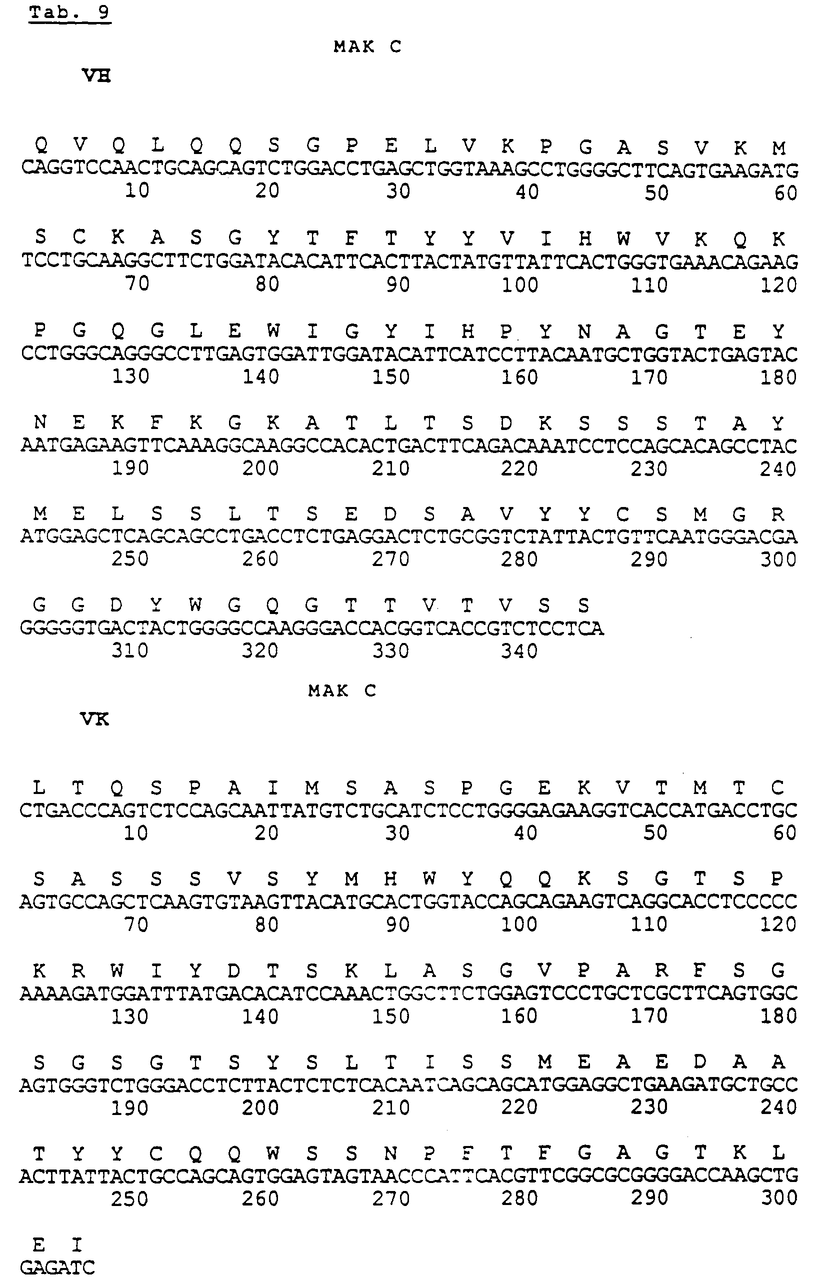

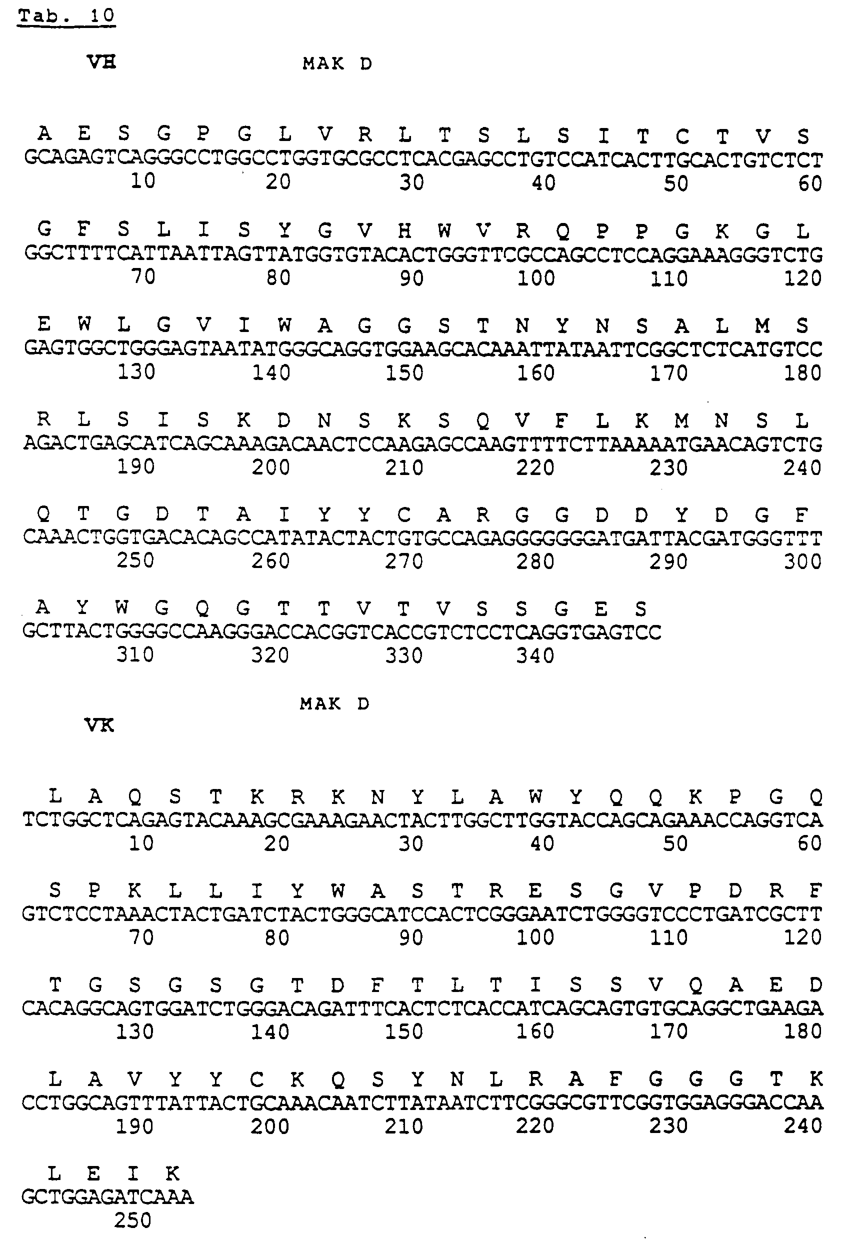

## Patentansprüche (Patentansprüche für folgende(n) Vertragsstaat(en): AT, BE, CH, DE, DK, FR, GB, IT, LI, LU, NL, SE)

1. Monoklonaler Antikörper, dadurch gekennzeichnet, daß er eine variable Domäne gemäß Tab. 7 enthält.

2. Monoklonaler Antikörper nach Anspruch 1, dadurch gekennzeichnet, daß er außerhalb der variablen Domäne ein humanes Antikörpergerüst enthält.

3. Monoklonaler Antikörper nach Anspruch 1, dadurch gekennzeichnet, daß die variable Domäne außerhalb der antigenbindenden Sequenzen humanen Ursprungs ist.

4. Monoklonaler Antikörper nach Anspruch 1, dadurch gekennzeichnet, daß er außerhalb der variablen Domäne ein murines Antikörpergerüst enthält.

5. Monoklonaler Antikörper nach Anspruch 1, 2, 3 oder 4 als Arzneimittel.

6. Monoklonaler Antikörper nach Anspruch 1, 2, 3 oder 4 als Diagnostikum.

7. Therapeutische Zubereitung, enthaltend monoklonale Antikörper nach Anspruch 1, 2, 3 oder 4 und ein inertes Trägermaterial.

8. Molekülkonstrukt, enthaltend als Anteil einen monoklonalen Antikörper oder reaktive Teile davon nach einem der Ansprüche 1, 2, 3 oder 4.

9. Molekülkonstrukt nach Anspruch 8, dadurch gekennzeichnet, daß Enzyme oder radioaktive Markierungen gekoppelt sind.

10. Molekülkonstrukt nach Anspruch 8, dadurch gekennzeichnet, daß Toxine oder katalytische Antikörper oder Kombinationen verschiedener V-Genspezifitäten oder MHC-Klasse I oder Klasse II Antigene oder cytolytische Komponenten gekoppelt sind.

## Patentansprüche (Patentansprüche für folgende(n) Vertragsstaat(en): ES)

1. Verfahren zur Herstellung eines monoklonalen Antikörpers, dadurch gekennzeichnet, daß die cDNA der variablen Domänen gemäß Tab. 7 in ein humanes Antikörpergerüst einligiert und in einem geeigneten Expressionssystem exprimiert wird.

2. Verfahren zur Herstellung eines monoklonalen Antikörpers, dadurch gekennzeichnet, daß die cDNA der variablen Domänen gemäß Tab. 7 in ein murines Antikörpergerüst einligiert und in einem geeigneten Expressionssystem exprimiert wird.

3. Verfahren zur Herstellung eines Arzneimittels, dadurch gekennzeichnet, daß nach Anspruch 1 oder 2 hergestellte monoklonale Antikörper mit üblichen Trägermaterialien gemischt werden.

4. Verfahren zur Herstellung von Diagnostika, dadurch gekennzeichnet, daß nach Anspruch 1 oder 2 hergestellte monoklonale Antikörper verwendet werden.

5. Verfahren zur Herstellung von Molekülkonstrukten, dadurch gekennzeichnet daß nach Anspruch 1 oder 2 hergestellte monoklonale Antikörper oder reaktive Teile davon an Enzyme, radioaktive Markierungen, Toxine katalytische Antikörper oder Kombinationen verschiedener V-Genspezifitäten oder MHC-Klasse I oder Klasse II Antigene oder cytolytische Komponenten gekoppelt sind.

## Patentansprüche (Patentansprüche für folgende(n) Vertragsstaat(en): GR)

1. Monoklonaler Antikörper, dadurch gekennzeichnet, daß er eine variable Domäne gemäß Tab. 7 enthält.

2. Monoklonaler Antikörper nach Anspruch 1, dadurch gekennzeichnet, daß er außerhalb der variablen Domäne ein humanes Antikörpergerüst enthält.

3. Monoklonaler Antikörper nach Anspruch 1, dadurch gekennzeichnet, daß die variable Domäne außerhalb der antigenbindenden Sequenzen humanen Ursprungs ist.

4. Monoklonaler Antikörper nach Anspruch 1, dadurch gekennzeichnet, daß er außerhalb der variablen Domäne ein murines Antikörpergerüst enthält.

5. Therapeutische Zubereitung, enthaltend monoklonale Antikörper nach Anspruch 1, 2, 3 oder 4 und ein inertes Trägermaterial.

6. Molekülkonstrukt, enthaltend als Anteil einen monoklonalen Antikörper oder reaktive Teile davon nach Anspruch 1, 2, 3 oder 4.

7. Molekülkonstrukt nach Anspruch 6, dadurch gekennzeichnet, daß Enzyme oder radioaktive Markierungen gekoppelt sind.

8. Molekülkonstrukt nach Anspruch 6, dadurch gekennzeichnet, daß Toxine oder katalytische Antikörper oder Kombinationen verschiedener V-Genspezifitäten oder MHC-Klasse I oder Klasse II Antigene oder cytolytische Komponenten gekoppelt sind.

## Claims (Claims for the following Contracting State(s): AT, BE, CH, DE, DK, FR, GB, IT, LI, LU, NL, SE)

1. A monoclonal antibody which comprises a variable domain as depicted in Tab. 7.

2. A monoclonal antibody as claimed in claim 1, which comprises a human antibody backbone outside the variable domain.

3. A monoclonal antibody as claimed in claim 1, wherein the variable domain outside the antigen-binding sequences is of human origin.

4. A monoclonal antibody as claimed in claim 1, which comprises a murine antibody backbone outside the variable domain.

5. A monoclonal antibody as claimed in claims 1, 2, 3 or 4 as a pharmaceutical.

6. A monoclonal antibody as claimed in claims 1, 2, 3 or 4 as a diagnostic agent.

7. A therapeutic preparation which comprises monoclonal antibodies as claimed in claims 1, 2, 3 or 4 and an inert carrier material.

8. A molecular construct which comprises, as a constituent, a monoclonal antibody, or reactive moieties thereof, as claimed in one of claims 1, 2, 3 or 4.

9. A molecular construct as claimed in claim 8, wherein enzymes or radioactive labels are coupled.

10. A molecular construct as claimed in claim 8, wherein toxins or catalytic antibodies or combinations of different V gene specificities or MHC class I or class II antigens or cytolytic components are coupled.

## Claims (Claims for the following Contracting State(s): ES)

1. A process for preparing a monoclonal antibody, which comprises ligating the cDNA for the variable domains as depicted in Tab. 7 into a human antibody backbone and expressing it in a suitable expression system.

2. A process for preparing a monoclonal antibody, which comprises ligating cDNA for the variable domains as depicted in Tab. 7 into a murine antibody backbone and expressing it in a suitable expression system.

3. A process for preparing a pharmaceutical, which comprises mixing monoclonal antibodies which have been prepared as claimed in claim 1 or 2 with customary carrier materials.

4. A process for preparing diagnostic agents, which comprises using monoclonal antibodies which have been prepared as claimed in claim 1 or 2.

5. A process for preparing molecular constructs, which comprises coupling monoclonal antibodies which have been prepared as claimed in claim 1 or 2, or reactive moieties thereof, to enzymes, radioactive labels, toxins, catalytic antibodies or combinations of different V gene specificities or MHC class I or class II antigens or cytolytic components.

## Claims (Claims for the following Contracting State(s): GR)

1. A monoclonal antibody which comprises a variable domain as depicted in Tab. 7.

2. A monoclonal antibody as claimed in claim 1, which comprises a human antibody backbone outside the variable domain.

3. A monoclonal antibody as claimed in claim 1, wherein the variable domain outside the antigen-binding sequences is of human origin.

4. A monoclonal antibody as claimed in claim 1, which comprises a murine antibody backbone outside the variable domain.

5. A therapeutic preparation which comprises monoclonal antibodies as claimed in claims 1, 2, 3 or 4 and an inert carrier material.

6. A molecular construct which comprises, as a constituent, a monoclonal antibody, or reactive moieties thereof, as claimed in one of claims 1, 2, 3 or 4.

7. A molecular construct as claimed in claim 6, wherein enzymes or radioactive labels are coupled.

8. A molecular construct as claimed in claim 6, wherein toxins or catalytic antibodies or combinations of different V gene specificities or MHC class I or class II antigens or cytolytic components are coupled.

## Revendications (Revendications pour l'(les) Etat(s) contractant(s) suivant(s): AT, BE, CH, DE, DK, FR, GB, IT, LI, LU, NL, SE)

1. Anticorps monoclonal, caractérisé en ce qu'il contient un domaine variable selon le tableau 7.

2. Anticorps monoclonal selon la revendication 1, caractérisé en ce que, outre le domaine variable, il comporte une charpente d'anticorps humain.

3. Anticorps monoclonal selon la revendication 1, caractérisé en ce que, hormis les séquences de fixation à l'antigène, le domaine variable est d'origine humaine.

4. Anticorps monoclonal selon la revendication 1, caractérisé en ce que, outre le domaine variable, il comporte une charpente d'anticorps murin.

5. Anticorps monoclonal selon la revendication 1, 2, 3 ou 4, en tant que médicament.

6. Anticorps monoclonal selon la revendication 1, 2, 3 ou 4, en tant qu'agent de diagnostic.

7. Composition thérapeutique, contenant des anticorps monoclonaux selon la revendication 1, 2, 3 ou 4 et un véhicule inerte.

8. Produit de construction moléculaire, contenant en tant que fragment un anticorps monoclonal ou des fragments réactifs de celui-ci, selon l'une des revendications 1, 2, 3 et 4.

9. Produit de construction moléculaire selon la revendication 8, caractérisé en ce que des enzymes ou des marqueurs radioactifs y sont couplés.

10. Produit de construction selon la revendication 8, caractérisé en ce que des toxines ou des anticorps catalytiques ou des associations de diverses spécificités de gène V, ou des antigènes de classe I ou de classe II du CMH (complexe majeur d'histocompatibilité), ou des composants cytolytiques y sont couplés.

## Revendications (Revendications pour l'(les) Etat(s) contractant(s) suivant(s): ES)

1. Procédé pour la production d'un anticorps monoclonal, caractérisé en ce que l'ADNc des domaines variables selon le tableau 7 est ligaturé dans une charpente d'anticorps humain, et exprimé dans un système d'expression approprié.

2. Procédé pour la production d'un anticorps monoclonal, caractérisé en ce que l'ADNc des domaines variables selon le tableau 7 est ligaturé dans une charpente d'anticorps murin, et exprimé dans un système d'expression approprié.

3. Procédé pour la fabrication d'un médicament, caractérisé en ce que l'on mélange des anticorps monoclonaux, produits selon la revendication 1 ou 2, avec des véhicules usuels.

4. Procédé pour la fabrication d'agents de diagnostic, caractérisé en ce que l'on utilise des anticorps monoclonaux produits selon la revendication 1 ou 2.

5. Procédé pour la production de produits de construction moléculaires, caractérisé en ce que des anticorps monoclonaux produits selon la revendication 1 ou 2, ou des fragments réactifs de ceux-ci, sont couplés à des enzymes, des marqueurs radioactifs, des toxines, des anticorps catalytiques ou des associations de diverses spécificités de gène V ou des antigènes de classe I ou de classe II du CMH (complexe majeur d'histocompatibilité), ou des composants cytolytiques.

## Revendications (Revendications pour l'(les) Etat(s) contractant(s) suivant(s): GR)

1. Anticorps monoclonal, caractérisé en ce qu'il contient un domaine variable selon le tableau 7.

2. Anticorps monoclonal selon la revendication 1, caractérisé en ce que, outre le domaine variable, il comporte une charpente d'anticorps humain.

3. Anticorps monoclonal selon la revendication 1, caractérisé en ce que, hormis les séquences de fixation à l'antigène, le domaine variable est d'origine humaine.

4. Anticorps monoclonal selon la revendication 1, caractérisé en ce que, outre le domaine variable, il comporte une charpente d'anticorps murin.

5. Composition thérapeutique, contenant des anticorps monoclonaux selon la revendication 1, 2, 3 ou 4 et un véhicule inerte.

6. Produit de construction moléculaire, contenant en tant que fragment un anticorps monoclonal ou des fragments réactifs de celui-ci, selon la revendication 1, 2, 3 ou 4.

7. Produit de construction moléculaire selon la revendication 6, caractérisé en ce que des enzymes ou des marqueurs radioactifs y sont couplés.

8. Produit de construction selon la revendication 6, caractérisé en ce que des toxines ou des anticorps catalytiques ou des associations de diverses spécificités de gène V, ou des antigènes de classe I ou de classe II du CMH (complexe majeur d'histocompatibilité), ou des composants cytolytiques y sont couplés.
